# EUROPEAN PATENT SPECIFICATION

(11) **EP 0 888 364 B1**
(45) Date of publication and mention of the grant of the patent: **06.02.2002**
(21) Application number: 97908576.8
(22) Date of filing: 07.03.1997
(51) Int. Cl.: C07F 9/145, C07F 9/24

(54) **Process to prepare a multidentate phosphite compound**
Verfahren zur Herstellung einer mehrzahnigen Phosphit-Verbindung
Procédé de preparation d'un composé phosphite multidenté

(30) Priority: 15.03.1996 US 616747
(43) Date of publication of application: 07.01.1999
(73) Proprietor: DSM N.V., 6411 TE Heerlen (NL); E.I. DU PONT DE NEMOURS AND COMPANY, Wilmington Delaware 19898 (US)
(72) Inventor: SNIJDER, Carina, Sacha, NL-6137 JR Sittard (NL); TEUNISSEN, Antonius, Jacobus, Josephus, Maria, NL-6165 XM Geleen (NL); HANSEN, Carolina, Bernedette, NL-6136 BT Sittard (NL); SHAPIRO, Rafael, Wilmington, DE 19803 (US); GARNER, James, Michael, Wilmington, DE 19803 (US)
(74) Representative: Kleiborn, Paul Erik
(86) International application number: PCT/NL97/00115
(87) International publication number: WO 97/33892

(56) References cited:
- EP-A- 0 518 241
- WO-A-96/11182
- WO-A-96/22968
- US-A- 4 501 684

## Description

The invention relates to a process to prepare a multidentate phosphite compound represented by the general formula (1) 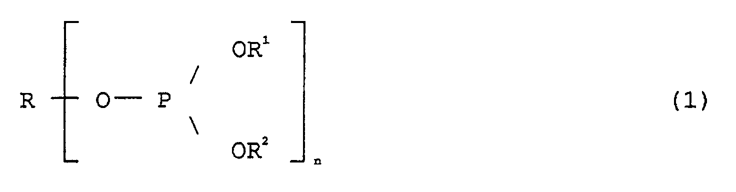 in which n is 2-6, R is an n-valent organic group and R¹ and R² are fused aromatic ring systems with 2 or more rings, which rings are substituted on the ortho position relative to the oxygen atom, only with hydrogen, provided substitution is possible. The phosphite compounds are prepared by first preparing a phosphorochloridite compound from R¹-OH and R²-OH alcohol compounds and a phosphorus chloride compound. In a subsequent contacting step the phosphorochloridite compound is contacted with an alcoholic compound according to R-(OH)ₙ to yield the phosphite compound.

Such a process is described in US-A-5235113. This patent publication describes the preparation of a phosphorochloridite by reacting 3,6-di-tert-butyl-2-naphthol dissolved in triethylamine and PCl₃ dissolved in toluene. A bidentate phosphite is subsequently prepared by contacing the phosphorochloridite compound with 2,2'-biphenyldiol in triethylamine.

Disadvantages of this known process include the difficulty in preparing the intermediate phosphorochloridite compound in a high yield when starting from alcohols (R¹OH and R²OH), in which the steric bulk of the molecule around the hydroxyl-functional group is not sufficiently large. The main product will then be a triorganophosphite compound, in which the organo-groups correspond with the starting alcohol compound. The formation of this compound can be explained by the less steric hinderance around the P-O bond of the phosphite, which makes it possible that three moles of alcohol compounds react with one mole of PCl₃.

There is a need for a process in which these phosphorochloridite compounds can be prepared in a high yield in order to be able to prepare the class of multidentate phosphite compounds according to formula (1) which presently are difficult to obtain.

This aim is achieved in that the phosphorochloridite, is prepared by performing the following two steps in a solvent:
a) contacting a compound with the general formula: 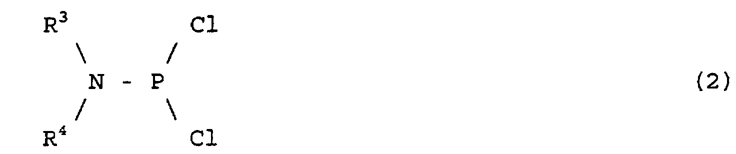 in which R³ and R⁴ are C₁-C₄ alkyl groups, with the R¹OH and R²OH compounds, and
b) contacting the resulting compound of step (a) which has the formula (3) : 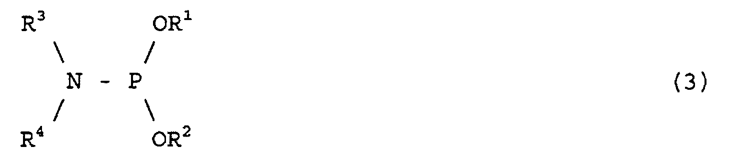 with HY, wherein Y represents a halogen atom. When using the process according to the invention it is possible to prepare the phosphorochloridite compound and thus the phosphite compound according to formula (1) in a high yield.

US-A-4501684 describes a process for preparing 2-napthyl phosphoramidites, i.e. a monodentate phosphite compound containing only one phosphorous atom.

A method for preparing phosphite compounds directly, in one step, from a dialkyl-N,N'-dialkylphosphoramite compound and a mono-alcohol is described in Synthesis, 1988, 2, 142. The resulting phosphite compound is however a monodentate phosphite compound. It appeared to be difficult to directly prepare a multidentate phosphite compound in an analogous method by starting from a compound, R(OH)ₙ, with more than one alcohol functionalities (n > 1).

Starting alcoholic compounds R¹OH and R²OH preferably have 10-30 carbon atoms and preferably comprise of between 2-4 fused aromatic rings. Examples are naphthol, anthranol and phenanthrol. All of the carbon atoms adjacent to the carbon atom on which the hydroxyl group is substituted, are substituted, if possible, only with hydrogen. The other carbon atoms of the fused rings may optionally be substituted with other groups, for example alkoxy, alkyl, amine and halogen groups. Preferably R¹ and R² are the same group. R¹ and R² are preferably 9-phenanthryl or 1-naphthyl groups.

Step (a) can be performed in the same manner as described in Synthesis, 1988, 2, 142-144. This article describes the preparation of alkyl and aryl di-tert-butyl phosphates using di-tert-butyl N,N-diethylphosphoramidite as an intermediate compound. This intermediate compound is analoguous to the compound as described by formula (3).

The compound according to formula (2) can be obtained by the methods known to the man skilled in the art, for example on an analogous manner as described in the above article.

R³ and R⁴ are C₁-C₄ alkyl, for example methyl, ethyl, propyl, butyl or tert-butyl. Preferably R³ and R⁴ are the same group.

In Step (a) the compound according to formula (2) is contacted in a suitable solvent with the R¹OH and R²OH, preferably in the presence of a base. Examples of bases include organic bases, for example a trialkylamine with 2 to 12 carbon atoms. Examples of suitable solvents are for example ethers, for example diethyl ether, dioxane or tetrahydrofuran or aromatic solvents for example benzene or toluene. Step (a) is preferably performed at a temperature between -80 and 60 °C, or more preferably about room temperature.

The concentration of the compound according to formula (2) is preferably between 0.01 and 5 mol/l. The molar ratio of R¹OH and R²OH and the compound (2) is preferably stoichiometric. Other ratio's are possible, but a greater effort is then needed to purify the product.

In Step (b) the Y of HY can be F, Cl, Br or I. A preferred HY is HCl. HY can be present in a gaseous form or dissolved in a solvent, for example an ether, for example diethyl ether, dioxane or tetrahydrofuran or aromatics, for example benzene or toluene. The temperature in step (b) is preferably between -80 and 60°C. The molar ratio of HCl and compound (3) is preferably between 0.8 and 5.

The step (b) phosphorochloridite reaction product, is generally obtained dissolved in the reaction mixture as obtained in step (b) having R³R⁴NH.HCl salt present. It can be advantageous to separate the salt from the phosphorochloridite compound before using this compound further. Separation can be performed by, for example, filtration of the reaction mixture.

The thus obtained phosphorochloridite compound is subsequently contacted with an alcoholic compound according to R-(OH)ₙ in which R is the n-valent organic group of formula (1) and n is 2-6.

The conditions for such a contacting in order to prepare the phosphite compound are generally known and are for example described in the aforementioned US-A-5235113. Generally the contacting is performed in a suitable solvent, for example the solvents as mentioned as possible solvents for step (a). Preferably the contacting is performed in the presence of a base, for example an organic base, for example alkylamine for example triethylamine. Temperature is preferably between -80 and 100°C.

The reaction mixture obtained in step (b), comprising the phosphorochloridite compound and the R³R⁴NH.HCl salt may be used in the preparation of the multidentate phosphite compound. Preferably the phosphorochloridite compound is first separated from the R³R⁴NH.HCl salt before preparing the phosphite compound. This is especially preferred when the R-(OH)ₙ compound is not very reactive. Separation may be for example performed by filtration.

The n-valent group R can be any organic bridging group which are commonly known for multidentate phosphite compounds, for example those groups which are described in US-A-5235113, EP-A-214622 or WO-A-9518089.

Preferably the n-valent group has at least two carbon atoms. The n-valent group preferably has less than 40 carbon atoms. The n-valent group may be alkylene groups or divalent aromatic groups. Examples of possible alkylene groups are ethylene, trimethylene, tetramethylene or pentamethylene. Examples of alcohols according to R-(OH)ₙ, which are the building blocks of the n-valent organic group are 2,5-di-t-butylhydroquinone, 2,5-di-t-amylhydroquinone, 2,5-dimethylhydroquinone, 4,6-di-t-butylresorcinol, 4,4'-isopropylidenebisphenol, 4,4'-methylenebis(2-methyl-6-t-butylphenol), 4,4'-oxobis(2-methyl-6-isopropylphenol), 4,6'-butylidenebis (3-methyl-6-t-butylphenol), 2,2'-biphenyldiol, 3,3',5,5'-tetramethyl-2,2'-biphenyldiol, 3,3',5,5'-tetra-t-butyl-2,2'-biphenyldiol, 3,3'-dimethoxy-5,5'-dimethyl-2,2'-biphenyldiol, 3,3'-di-t-butyl-5,5'-dimethoxy-2,2'-biphenyldiol, 3,3'-di-t-butyl-5,5'-dimethyl-2,2'-biphenyldiol, 2,2'-methylenebis(4-methyl-6-t-butylphenol), 2,2'-methylenebis(4-ethyl-6-t-butylphenol), 2,2'-thiobis(4-methyl-6-t-butylphenol), 2,2'-thiobis(4-t-butyl-6-methylphenol), 2,2'-thiobis(4,6-di-t-butylphenol), 1,1'-thiobis(2-naphthol), catechol, 2,3-dihydroxynapthalene, 1,8-dihydroxynaphthalene, 1,4-dihydroxynaphthalene, 1,3,5-trihydroxybenzene, 1,1'-methylenebis (2-naphthol), 1,1'-di-2-naphthol, 10,10'-di-9-phenanthrol, ethyleneglycol, 1,3-propanediol, 1,2-butanediol, 1,4-butanediol, pentaerythritol, trans-1,2-cyclohexanediol, cis-1,2-cyclohexanediol, cis-1,2-cyclohexanedimethanol, cis-1,2-cyclododecanediol or the like.

The invention also relates to bidentate phosphite compounds according to formula (1): 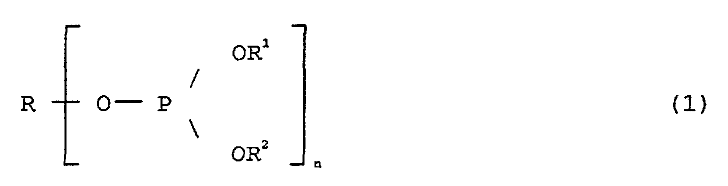 in which n is 2-6, R is an n-valent organic group and R¹ and R² are fused aromatic ring systems with 2 or more rings, which rings are substituted on the ortho position relative to the oxygen atom only with hydrogen, provided substitution is possible.

A preferred and novel class of bidentate phosphite compounds according to formula (1) have a group R according to the following formula (4): 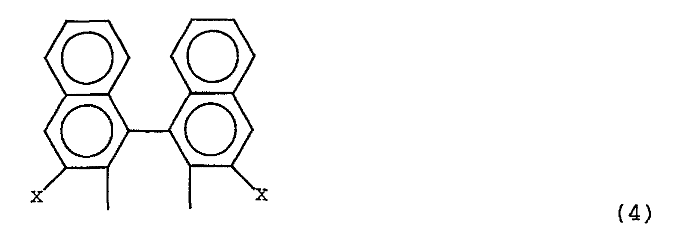 in which X is hydrogen or an organic group and preferably both X are an organic group and more preferably an alkyl group, an aryl group, a triarylsilyl group, a trialkylsilyl group, a carboalkoxy group, a carboaryloxy group, an aryloxy group, an alkoxy group, an alkylcarbonyl group, an arylcarbonyl group, an amide or a nitrile group. The invention is also directed to this novel bidentate phosphite ligand.

The alkyl group is preferably a C₁-C₁₀ alkyl group, for example methyl, ethyl, propyl, isopropyl, butyl, tert-butyl, isobutyl, pentyl or hexyl. An example of a triarylsilyl group is triphenylsilyl and examples of a trialkylsilyl group are trimethylsilyl and triethylsilyl. Preferred aryl groups have 6 to 20 carbon atoms, for example benzyl, tolyl, naphthyl or phenanthryl. Preferred aryloxy groups have 6 to 12 carbon atoms, for example phenoxy. Preferred alkoxy groups have 1 to 10 carbon atoms, for example methoxy, ethoxy, tert-butoxy or isopropoxy. Preferred alkylcarbonyl groups have 2 to 12 carbon atoms, for example methylcarbonyl, tert-butylcarbonyl. Preferred arylcarbonyl groups have 7 to 13 carbon atoms, for example phenylcarbonyl.

X is most preferably a carboalkoxyl or carboaryloxy groups, -CO-O-R³, in which R³ is an alkyl group with 1 to 20 carbon atoms or an aryl group with 6-12 carbon atoms. Examples of suitable R groups are methyl, ethyl, propyl, isopropyl, n-butyl, tert-butyl, isobutyl, phenyl, tolyl. Even more preferably both X's are substituted with the same carboalkoxyl group.

R¹ and R² are preferably 9-phenanthryl or 1-naphthyl groups.

The phosphite compounds having a bridging group R according to formula (4) as described above can be advantageously used as part of a homogeneous catalyst system also comprising rhodium. Such a catalyst system is preferably used for the hydroformylation reaction of an unsaturated organic compound and especially of an internally unsaturated organic compound to a terminal aldehyde organic compound. High reaction rates and high selectivities to linear aldehyde (terminal aldehyde) products can be achieved when using such catalyst system.

For example it has been found that when using such a catalyst system the reaction of a C₁-C₆ alkyl 3-pentenoate ester to the C₁-C₆ alkyl 5-formylvalerate ester proceeds with a high selectivity, yield and reaction rate when compared to state of the art processes, such as form example described in WO-A-9518089. The invention is also directed to a process to prepare C₁-C₆ alkyl 5-formylvalerate esters. Preferably the alkyl is methyl or ethyl.

Preferably the multidentate phosphite compound according to formula (1) as obtained by the process of the invention is used as a polymer stabilizer.

Preferably the multidentate phosphite compound according to formula (1) as obtained by the process of the invention is used as a fire retarding additive.

Preferably the multidentate phosphite compound as obtained by the process of the invention is used as part of a homogeneous catalyst system also comprising a metal of Groups 8-10. The homogeneous catalyst system can be used for the isomerization of olefins. More preferably the homogeneous catalyst system is used in the hydroformylation of an ethylenically unsaturated organic compound to a terminal aldehyde compound. When used as a hydroformylation catalyst the group 8-10 metal is preferably rhodium or iridium and most preferably rhodium. The molar ratio of multidentate phosphite ligand to rhodium is generally from about 0.5 to 100 and preferably from 1 to 10 and most preferably less than 1.2 (mol ligand/mol rhodium). It has been found that by using a slight excess of ligand, the ligand degradation can be reduced. Preferably the unsaturated compound is an pentenoic acid, its corresponding ester or pentenitrile. The resulting 5-formylvaleric acid, its ester or nitrile are important intermediates in a process to prepare precursors for Nylon-6 and Nylon-6.6.

The hydroformylation reaction is preferably performed as described below.

The concentration of rhodium in the reaction mixture may vary from 1 to 5000 ppm rhodium. Preferably, the concentration is between 50 and 1000 ppm.

The reaction mixture may serve as a solvent, so that as a rule the addition of an additional solvent is not necessary. The reaction mixture is a mixture of the reactants of the hydroformylation, for example the unsaturated organic compound, the aldehyde and/or by-products formed, in particular the by-products with high boiling temperatures. If an additional solvent is added, a saturated hydrocarbon, for example naphtha, kerosine, mineral oil or cyclohexane, or an aromatic compound, for example toluene, benzene, xylene, or an ether, for example diphenylether, tetrahydrofuran, or a ketone, for example cyclohexanone, or a nitrile, for example benzonitrile, texanol® or tetraglyme® (Union Carbide), is suitable for use as additional solvent. A mixture of two or more of these compounds is also suitable for use as additional solvent.

The temperature is generally between room temperature and 200°C, preferably between 50°C and 150°C. The pressure is generally between 0.1 MPa and 20 MPa, preferably between 0.15 MPa and 10 MPa and most preferably between 0.2 MPa and 1 MPa.

The molar ratio of hydrogen and carbon monoxide is generally between 10:1 and 1:10, preferably between 6:1 and 1:2.

The hydroformylation reaction can be carried out in a gas/liquid contactor known to a person skilled in the art. Examples of suitable reactors are a bubble column, screen-plate column or a gas-liquid agitated reactor.

The invention shall be elucidated with the following non-limiting examples.

### Example I

3.88 g (20 mmol) 9-phenanthrol was dissolved in 250 ml toluene and the water was removed by azeotropic distillation. Subsequently, 2.3 g triethylamine and 1.74 g (10 mmol) diethylamino-phosphorous dichloride were added at room temperature while stirring. In this way diethylamino diphenanthrene phosphite was synthesized (³¹P NMR δ 138.7 ppm). To obtain diphenanthrene phosphorous chloride 22 ml of a 1 M HCl solution in diethyl ether was added (³¹P NMR δ 161.4 ppm). The reaction mixture was filtered and to the filtrate 3 g (30 mmol) triethyl amine and 1.98 g dimethyl 2,2'-dihydroxy-1,1'-binaphthalene-3,3'-dicarboxylate were added. After stirring for 10 minutes the reaction was completed and NEt₃.HCl was removed by filtration (Et = ethyl). The solvent was removed and the product was purified by crystallization from acetonitril/toluene (³¹P NMR δ 126.6 ppm). The yield of Compound 1 was 90%.

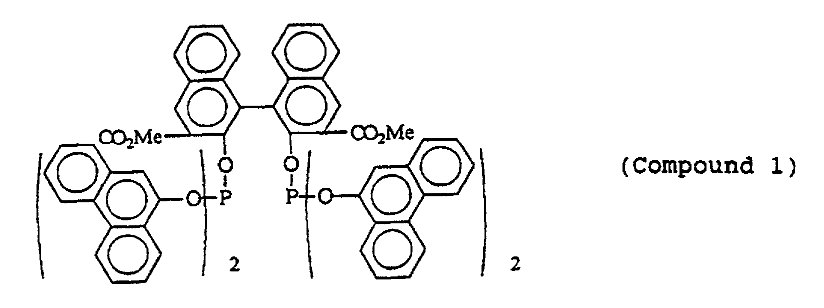

### Comparative Experiment A

Compound 1 was also intended to be prepared by the synthetic route as described in Example 10 of US-A-5235113 starting from 9-phenanthrol. It was however not possible to obtain the phosphorochloridite intermediate in a high yield (about 5%). Mainly tri(9-phenanthryl)phosphite was obtained. The 5% of the desired product could not be isolated easily. This experiment illustrates that it is difficult to prepare the Compound 1 by the prior art synthetic route.

### Example II

A 150 ml Hastelloy-C steel autoclave (Parr) was filled under nitrogen with 5.8 mg Rh(acac)(CO)₂ (acac = acetylacetonate) (4.8 x 10⁻⁵ mol), 14.0 x 10⁻⁵ mol of Compound 1 as ligand (ligand/rhodium ratio (L/Rh) = 2.9 mol/mol) and 60 ml of toluene. Hereafter, the autoclave was closed and purched with nitrogen. Next, the autoclave was brought to a pressure of 1 MPa using carbon monoxide/hydrogen (1:1) and heated to 90°C in approx. 30 min. Subsequently a mixture of 7.44 g (65 mmol) freshly distilled methyl 3-pentenoate and 1.2 gram of nonane topped up to 15 ml with toluene was injected into the autoclave. The composition of the reaction mixture was determined by gas chromatography. After 7 hours of reaction a 90.1% conversion was determined. The selectivity to methyl 5-formylvalerate was 75.1%. The molar ratio of methyl 5-formylvalerate and the sum of methyl 3- and methyl 4-formylvalerate (n/b ratio) was 9.3, and the hydrogenation to methyl valerate was 5.7%.

### Example III

Example II was repeated in which the L/Rh was 3.1 with a ligand according to: 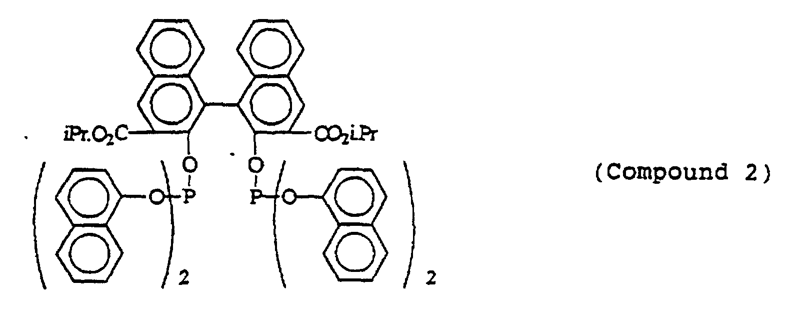 The conversion was 81.8% and the selectivity to methyl 5-formylvalerate was 84.6%.

### Example IV

Example I was repeated using as R-(OH)ₙ compound di-isopropyl 2,2'dihydroxy-1,1'-binaphthalene-3,3'-dicarboxylate and as R¹(OH) starting compound 9-phenanthrol. A compound according to: 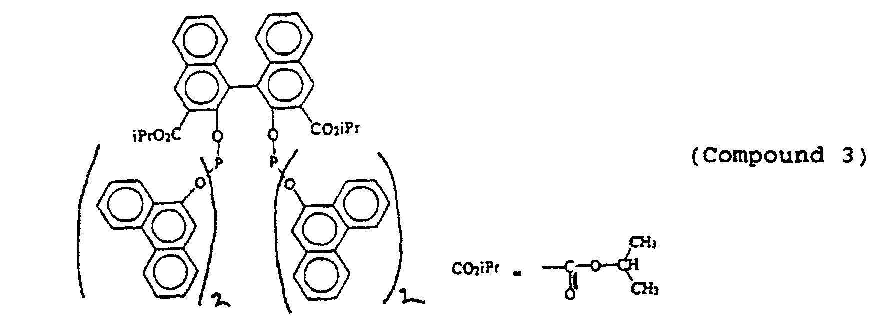 was obtained as a slightly yellow coloured powder in a 85 % yield.

### Example V

Example I was repeated using as R-(OH)ₙ compound pentaerythritol and as R¹(OH) starting compound 1-naphthol. A compound according to: 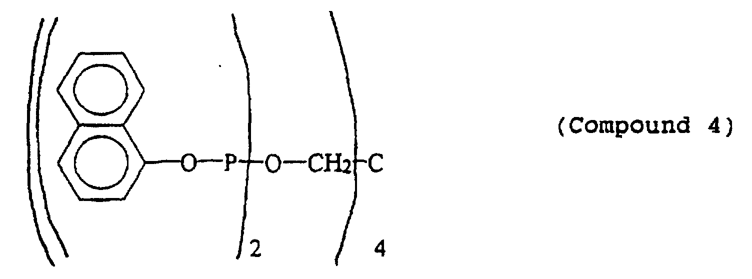 was obtained as a yellow coloured oil in a 80 % yield.

### Example VI

Example I was repeated using as R-(OH)ₙ compound diethyl 2,2'-dihydroxy-1,1'-binaphthalene-3,3'-dicarboxylate and as R¹(OH) starting compound 4-chloro-1-naphthol. The yield was about 90%.

## Claims

1. Process to prepare a multidentate phosphite compound represented by the general formula (1) 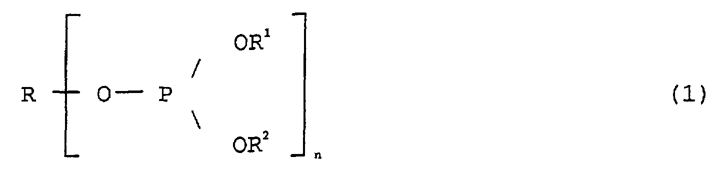 in which n is 2-6, R is an n-valent organic group and R¹ and R² are fused aromatic ring systems with 2 or more rings, which rings are substituted on the ortho position relative to the oxygen atom only with hydrogen, by first preparing a phosphorochloridite compound from a R¹-OH and R²-OH alcohol compound and a phosphorus chloride compound and subsequently contacting the phosphorochloridite compound with an alcoholic compound according to R - (OH)ₙ, **characterized in that** the phosphorochloridite is prepared by performing the two steps in a solvent:
a) contacting a compound with the general formula: 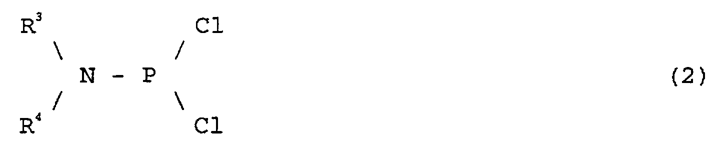 in which R³ and R⁴ are C₁-C₄ alkyl groups, with the R¹OH and R²OH compounds, and
b) contacting the resulting compound of step (a) which has the formula (3): 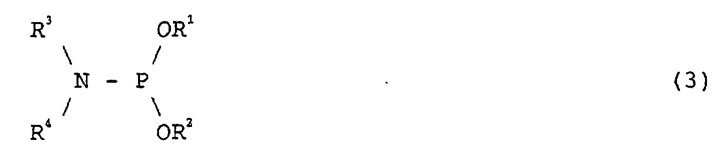 with HY, wherein Y represents a halogen atom.

2. Process according to claim 1, **characterized in that** HY is HCl.

3. Process according to any one of claims 1-2, **characterized in that** R³ and R⁴ are the same alkyl group.

4. Process according to any one of claims 1-3, **characterized in that** the HCl is dissolved in a solvent when contacting with the compound according to formula (3).

5. Process according to any one of claims 1-4, **characterized in that** gaseous HCl is contacted with the compound according to formula (3) present in the solvent.

6. Process according to any one of claims 1-5, **characterized in that** the temperature of step (a) and (b) is between - 80°C and 60°C.

7. Process according to any one of claims 1-6, **characterized in that** the phosphorochloridite compound is separated from the precipitated R³R⁴NH.HCl salt, which salt is also formed in the process, of the reaction mixture which results from the process.

8. Process according to any one of claims 1-7, **characterized in that** R¹-OH and R²-OH are one of the alcohols including naphthol, anthranol or phenanthrol.

9. Process according to any one of claims 1-8, **characterized in that** group R has between 2 to 40 carbon atoms.

10. Bidentate phosphite compound according to the following general formula (1): 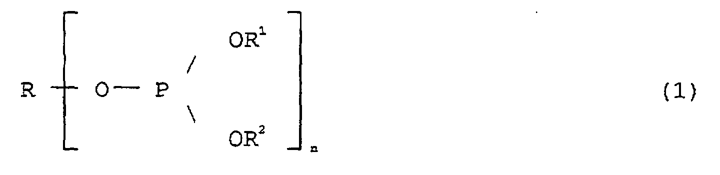 in which n is 2-6, R is an n-valent organic group and R¹ and R² are fused aromatic ring systems with 2 or more rings, which rings are substituted on the ortho position relative to the oxygen only with hydrogen.

11. Bidentate phosphite compounds according to claim 10, **characterized in that**, the bidentate phosphite compound is represented by the following general formula: 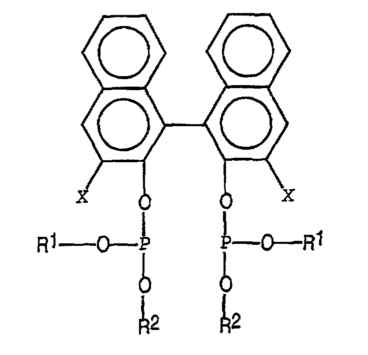 in which X is hydrogen or an organic group and R¹ and R² are fused aromatic ring systems with 2 or more rings, which rings are substituted on the ortho position relative to the oxygen only with hydrogen.

12. Bidentate phosphite compound according to claim 11, **characterized in that** X is an alkyl group, an aryl group, a triaryl silyl group, a trialkylsilyl group, a carboalkoxy group, a carboaryloxy group, an aryloxy group, an alkoxy group, an alkylcarbonyl group, an arylcarbonyl group, a nitrile group or an amide group.

13. Bidentate phosphite compound according to claim 12, **characterized in that** X is a carbo alkoxyl group or carboaryloxy group according to -CO - O - R³, in which R³ is an alkyl group with 1 to 20 carbon atoms or an aryl group with 6-12 carbon atoms.

14. Bidentate phosphite compound according to any one of claims 10-13, **characterized in that** R¹ and R² are 1-naphthyl or 9-phenanthryl groups.

15. Process to prepare a bidentate phosphite compound according to claims 10-14 by using the process according to any one of claims 1-9.

16. Homogeneous catalyst system comprising a metal of Groups 8-10 and a multidentate phosphite compound as obtained by the process according to any one of claims 1-9 or 15.

17. Homogeneous catalyst system according to claim 16, **characterized in that** the group 8-10 metal is rhodium.

18. Homogeneous catalyst system according to claims 16-17, **characterized in that** the phosphite to Group 8-10 metal molar ratio is between about 1 and 1.2.

19. Use of the homogeneous catalyst system of any of claims 16-18 in the hydroformylation of an ethylenically unsaturated organic compound to an aldehyde compound.

20. Use according to claim 19, **characterized in that** the ethylenically unsaturated organic compound is a C₁-C₆ alkyl 3-pentenoate.

## Patentansprüche

1. Verfahren zur Herstellung einer mehrzähnigen Phosphitverbindung, dargestellt durch die allgemeine Formel (1) 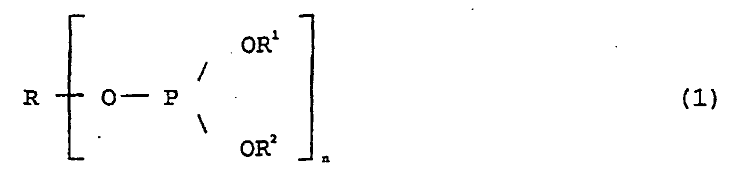 worin n 2 bis 6 ist, R ein n-wertiger organischer Rest ist und R¹ und R² miteinander kondensierte aromatische Ringsysteme mit zwei oder mehreren Ringen sind, wobei die Ringe in der ortho-Position relativ zu dem Sauerstoffatom nur mit Wasserstoff substituiert sind,
durch anfängliches Herstellen einer Phosphorochloriditverbindung aus einer Alkoholverbindung R¹-OH und R²-OH und einer Phosphorchloridverbindung und anschließendes Kontaktieren der Phosphorochloriditverbindung mit einer alkoholischen Verbindung gemäß R-(OH)ₙ, **dadurch gekennzeichnet, daß** das Phosphorochloridit durch Durchführen der zwei Schritte in einem Lösungsmittel hergestellt wird:
a) Kontaktieren einer Verbindung der allgemeinen Formel: 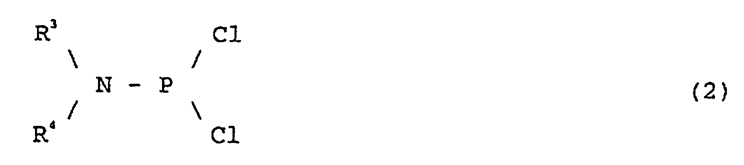 worin R³ und R⁴ C₁-C₄-Alkylreste sind, mit den Verbindungen R¹OH und R²OH und
b) Kontaktieren der resultierenden Verbindung aus Schritt (a), welche die Formel (3) aufweist: 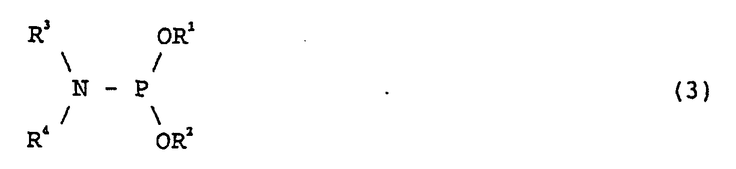 mit HY, worin Y ein Halogenatom darstellt.

2. Verfahren nach Anspruch 1, **dadurch gekennzeichnet, daß** HY HCl ist.

3. Verfahren nach Anspruch 1 oder 2, **dadurch gekennzeichnet, daß** R³ und R⁴ die gleichen Alkylreste sind.

4. Verfahren nach einem der Ansprüche 1 bis 3, **dadurch gekennzeichnet, daß** HCl in einem Lösungsmittel gelöst wird, wenn es mit der Verbindung gemäß Formel (3) kontaktiert wird.

5. Verfahren nach einem der Ansprüche 1 bis 4, **dadurch gekennzeichnet, daß** gasförmiges HCl mit der Verbindung gemäß Formel (3), welche in dem Lösungsmittel vorhanden ist, kontaktiert wird.

6. Verfahren nach einem der Ansprüche 1 bis 5, **dadurch gekennzeichnet, daß** die Temperatur in Schritt (a) und (b) zwischen -80°C und 60°C liegt.

7. Verfahren nach einem der Ansprüche 1 bis 6, **dadurch gekennzeichnet, daß** die Phosphorochloriditverbindung von dem ausgefällten R³R⁴NH·HCl Salz, welches auch in dem Verfahren gebildet wird, von dem Reaktionsgemisch, welches aus dem Verfahren resultiert, getrennt wird.

8. Verfahren nach einem der Ansprüche 1 bis 7, **dadurch gekennzeichnet, daß** R¹-OH und R²-OH einer der Alkohole, einschließlich Naphthol, Anthrol oder Phenanthrol, sind.

9. Verfahren nach einem der Ansprüche 1 bis 8, **dadurch gekennzeichnet, daß** der Rest R zwischen 2 bis 40 Kohlenstoffatome aufweist.

10. Zweizähnige Phosphitverbindung gemäß der folgenden allgemeinen Formel (1): 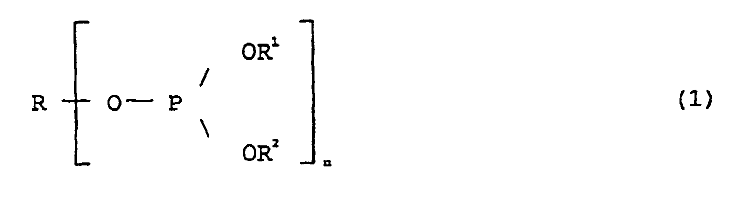 worin n 2 bis 6 ist, R ein n-wertiger organischer Rest ist und R¹ und R² miteinander kondensierte aromatische Ringsysteme mit zwei oder mehreren Ringen sind, wobei die Ringe in der ortho-Position relativ zu dem Sauerstoff nur mit Wasserstoff substituiert sind.

11. Zweizähnige Phosphitverbindung nach Anspruch 10, **dadurch gekennzeichnet, daß** die zweizähnige Phosphitverbindung durch die folgende allgemeine Formel dargestellt ist: 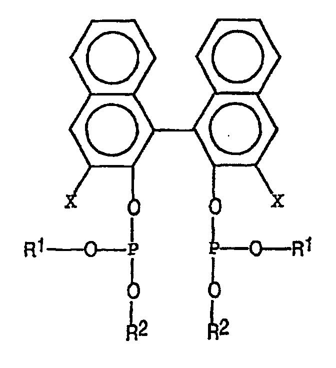 worin X Wasserstoff oder ein organischer Rest ist und R¹ und R² miteinander kondensierte aromatische Ringsysteme mit zwei oder mehreren Ringen sind, wobei die Ringe in der ortho-Position relativ zu dem Sauerstoff nur mit Wasserstoff substituiert sind.

12. Zweizähnige Phosphitverbindungen nach Anspruch 11, **dadurch gekennzeichnet, daß** X ein Alkylrest, ein Arylrest, ein Triarylsilylrest, ein Trialkylsilylrest, ein Carboalkoxyrest, ein Carboaryloxyrest, ein Aryloxyrest, ein Alkoxyrest, ein Alkylcarbonylrest, ein Arylcarbonylrest, eine Nitrilgruppe oder eine Amidgruppe ist.

13. Zweizähnige Phosphitverbindungen nach Anspruch 12, **dadurch gekennzeichnet, daß** X ein Carboalkoxylrest oder ein Carboaryloxyrest gemäß -CO-O-R³ ist, worin R³ ein Alkylrest mit 1 bis 20 Kohlenstoffatomen oder ein Arylrest mit 6 bis 12 Kohlenstoffatomen ist.

14. Zweizähnige Phosphitverbindung nach einem der Ansprüche 10 bis 13, **dadurch gekennzeichnet, daß** R¹ und R² 1-Naphthyl- oder 9-Phenanthrylgruppen sind.

15. Verfahren zur Herstellung einer zweizähnigen Phosphitverbindung nach einem der Ansprüche 10 bis 14 unter Verwendung des in einem der Ansprüche 1 bis 9 beschriebenen Verfahrens.

16. Homogenkatalysatorsystem, umfassend ein Metall der Gruppen 8 bis 10 und eine mehrzähnige Phosphitverbindung, wie durch das Verfahren nach einem der Ansprüche 1 bis 9 oder 15 erhalten.

17. Homogenkatalysatorsystem nach Anspruch 16, **dadurch gekennzeichnet, daß** das Metall der Gruppen 8 bis 10 Rhodium ist.

18. Homogenkatalysatorsystem nach Anspruch 16 oder 17, **dadurch gekennzeichnet, daß** das Molverhältnis des Phosphits zu dem Metall der Gruppen 8 bis 10 zwischen etwa 1 und 1,2 beträgt.

19. Verwendung des Homogenkatalysatorsystems nach einem der Ansprüche 16 bis 18 in der Hydroformylierung einer ethylenisch ungesättigten organischen Verbindung zu einer Aldehydverbindung.

20. Verwendung nach Anspruch 19, **dadurch gekennzeichnet, daß** die ethylenisch ungesättigte organische Verbindung ein C₁-C₆-Alkyl-3-pentenoat ist.

## Revendications

1. Procédé de préparation d'un composé phosphite multidenté représenté par la formule générale (1) 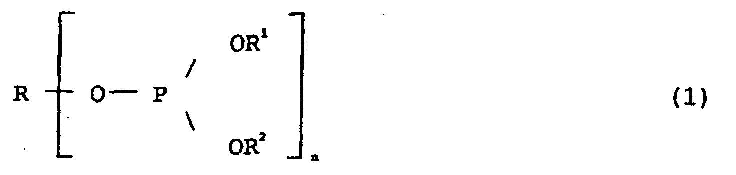 dans laquelle n vaut 2 à 6, R est un groupe organique n-valent et R¹ et R² sont des systèmes à noyau aromatique enchaîné comprenant au moins 2 noyaux, lesquels noyaux ne sont substitués en position ortho par rapport à l'atome d'oxygène que par un hydrogène, consistant à préparer tout d'abord un composé phosphorochloridite à partir d'un composé alcool R¹-OH et R²-OH et un composé chlorure de phosphore, puis en mettant le composé phosphorochloridite au contact d'un composé alcoolique en conformité avec R-(OH)ₙ, **caractérisé en ce que** le phosphorochloridite est préparé en conduisant dans un solvant les deux étapes consistant à :
a) mettre en contact un composé ayant la formule générale : 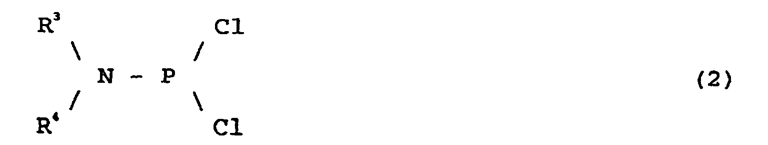 dans laquelle R³ et R⁴ sont des groupes alkyle en C₁ à C₄, avec les composés R¹OH et R²OH, et
b) mettre en contact le composé résultant de l'étape (a) qui a la formule (3): 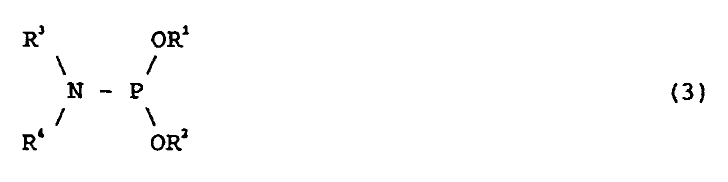 avec HY, où Y représente un atome d'halogène.

2. Procédé selon la revendication 1, **caractérisé en ce que** HY est HCI.

3. Procédé selon l'une quelconque des revendications 1 ou 2, **caractérisé en ce que** R³ et R⁴ sont le même groupe alkyle.

4. Procédé selon l'une quelconque des revendications 1 à 3, **caractérisé en ce que** le HCl est dissous dans un solvant lorsqu'il est mis en contact avec le composé en conformité avec la formule (3).

5. Procédé selon l'une quelconque des revendications 1 à 4, **caractérisé en ce que** le HCl gazeux est mis en contact avec le composé en conformité avec la formule (3) présent dans le solvant.

6. Procédé selon l'une quelconque des revendications 1 à 5, **caractérisé en ce que** la température de l'étape (a) et de l'étape (b) est comprise entre -80°C et 60°C.

7. Procédé selon l'une quelconque des revendications 1 à 6, **caractérisé en ce que** le composé phosphorochloridite est séparé du sel R³R⁴NH.HCl précipité, lequel sel est également formé dans le procédé du mélange réactionnel qui résulte du procédé.

8. Procédé selon l'une quelconque des revendications 1 à 7, **caractérisé en ce que** R¹-OH et R²-OH sont l'un des alcools incluant le naphtol, l'anthranol ou le phénanthrol.

9. Procédé selon l'une quelconque des revendications 1 à 8, **caractérisé en ce que** le groupe R comporte entre 2 et 40 atomes de carbone.

10. Composé phosphite bidenté en conformité avec la formule générale (1) suivante : 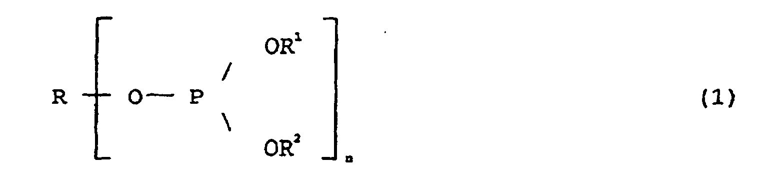 dans laquelle n vaut 2 à 6, R est un groupe organique n-valent et R¹ et R² sont des systèmes à noyau aromatique enchaîné comprenant au moins 2 noyaux, lesquels noyaux ne sont substitués en position ortho par rapport à l'oxygène que par un hydrogène.

11. Composés phosphites bidentés selon la revendication 10, **caractérisés en ce que** le composé phosphite bidenté est représenté par la formule générale suivante : 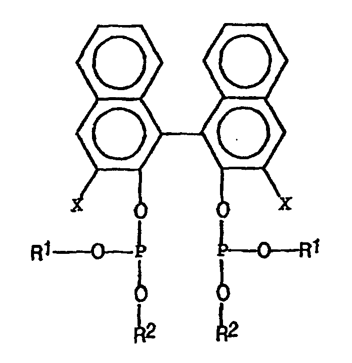 dans laquelle X est un hydrogène ou un groupe organique et R¹ et R² sont des systèmes à noyau aromatique enchaîné comprenant au moins 2 noyaux, lesquels noyaux ne sont substitués en position ortho par rapport à l'oxygène que par un hydrogène.

12. Composé phosphite bidenté selon la revendication 11, **caractérisé en ce que** X est un groupe alkyle, un groupe aryle, un groupe triarylsilyle, un groupe trialkylsilyle, un groupe carboalcoxy, un groupe carboaryloxy, un groupe aryloxy, un groupe alcoxy, un groupe alkylcarbonyle, un groupe arylcarbonyle, un groupe nitrile ou un groupe amide.

13. Composé phosphite bidenté selon la revendication 12, **caractérisé en ce que** X est un groupe carboalcoxyle ou un groupe carboaryloxy en conformité avec -CO-O-R³, dans lequel R³ est un groupe alkyle comportant 1 à 20 atomes de carbone ou un groupe aryle comportant 6 à 12 atomes de carbone.

14. Composé phosphite bidenté selon Tune quelconque des revendications 10 à 13, **caractérisé en ce que** R¹ et R² sont un groupe 1-naphtyle ou un groupe 9-phénanthrile.

15. Procédé de préparation d'un composé phosphite bidenté selon la revendication 10 à 14 en utilisant le procédé selon Tune quelconque des revendications 1 à 9.

16. Système catalyseur homogène comprenant un métal des groupes 8 à 10 et un composé phosphite multidenté tel qu'obtenu par le procédé selon Tune quelconque des revendications 1 à 9 ou 15.

17. Système catalyseur homogène selon la revendication 16, **caractérisé en ce que** le métal des groupes 8 à 10 est le rhodium.

18. Système catalyseur homogène selon les revendications 16 et 17, **caractérisé en ce que** le rapport molaire entre phosphite et métal des groupes 8 à 10 est compris entre environ 1 et 1,2.

19. Utilisation du système catalyseur homogène selon Tune quelconque des revendications 16 à 18 dans l'hydroformylation d'un composé organique éthyléniquement insaturé en un composé aldéhyde.

20. Utilisation selon la revendication 19, **caractérisé en ce que** le composé organique éthyléniquement insaturé est un 3-penténoate d'alkyle en C₁ à C₆.
